# EUROPEAN PATENT SPECIFICATION

(11) **EP 1 199 045 B1**
(45) Date of publication and mention of the grant of the patent: **11.06.2008**
(21) Application number: 01308879.4
(22) Date of filing: 19.10.2001
(51) Int. Cl.: A61B 17/32, A61F 9/007

(54) **Method for differentiating between burdened and cracked ultrasonically tuned blades**
Verfahren zum Unterscheiden zwischen verschmutzten und gebrochenen Ultraschall betriebenen Klingen
Procede permettant de differencier des lames usees et des lames cassees activees par des ultrasons

(30) Priority: 20.10.2000 US 241888 P; 14.08.2001 US 930104 P
(43) Date of publication of application: 24.04.2002
(60) Divisional application: 05076515.5
(73) Proprietor: ETHICON ENDO-SURGERY, Cincinnati, Ohio 45242 (US)
(72) Inventor: Friedman, Allan L., Cincinnati, OH 45237 (US); Donofrio, William T., Cincinnati, OH 45249 (US)
(74) Representative: Fisher, Adrian John

(56) References cited:
- EP-A- 0 359 217
- EP-A- 0 424 685
- US-A- 5 026 387
- US-A- 5 935 143
- US-A- 6 117 152

## Description

### BACKGROUND OF THE INVENTION

### 1, FIELD OF THE INVENTION

The present invention generally relates to ultrasonic surgical systems and, more particularly, to a method for differentiating between ultrasonically tuned blades which are broken or cracked and those which are gunked.

### 2. DESCRIPTION OF THE RELATED ART

It is known that electric scalpels and lasers can be used as a surgical instrument to perform the dual function of simultaneously effecting the incision and hemostatis of soft tissue by cauterizing tissues and blood vessels. However, such instruments employ very high temperatures to achieve coagulation, causing vaporization and fumes as well as splattering. Additionally, the use of such instruments often results in relatively wide zones of thermal tissue damage.

Cutting and cauterizing of tissue by means of surgical blades vibrated at high speeds by ultrasonic drive mechanisms is also well known. One of the problems associated with such ultrasonic cutting instruments is uncontrolled or undamped vibrations and the heat, as well as material fatigue resulting therefrom. In an operating room environment attempts have been made to control this heating problem by the inclusion of cooling systems with heat exchangers to cool the blade. In one known system, for example, the ultrasonic cutting and tissue fragmentation system requires a cooling system augmented with a water circulating jacket and means for irrigation and aspiration of the cutting site. Another known system requires the delivery of cryogenic fluids to the cutting blade.

It is known to limit the current delivered to the transducer as a means for limiting the heat generated therein. However, this could result in insufficient power to the blade at a time when it is needed for the most effective treatment of the patient. U.S. Patent No. 5,026,387 to *Thomas*. which is assigned to the assignee of the present application, discloses a system for controlling the heat in an ultrasonic surgical cutting and hemostasis system without the use of a coolant, by controlling the drive energy supplied to the blade. In the system according to this patent an ultrasonic generator is provided which produces an electrical signal of a particular voltage, current and frequency, e.g. 55,500 cycles per second. The generator is connected by a cable to a hand piece which contains piezoceramic elements forming an ultrasonic transducer. In response to a switch on the hand piece or a foot switch connected to the generator by another cable, the generator signal is applied to the transducer, which causes a longitudinal vibration of its elements. A structure connects the transducer to a surgical blade, which is thus vibrated at ultrasonic frequencies when the generator signal is applied to the transducer. The structure is designed to resonate at the selected frequency, thus amplifying the motion initiated by the transducer.

The signal provided to the transducer is controlled so as to provide power on demand to the transducer in response to the continuous or periodic sensing of the loading condition (tissue contact or withdrawal) of the blade. As a result, the device goes from a low power, idle state to a selectable high power, cutting state automatically depending on whether the scalpel is or its not in contact with tissue. A third, high power coagulation mode is manually selectable with automatic return to an idle power level when the blade is not in contact with tissue. Since the ultrasonic power is not continuously supplied to the blade, it generates less ambient beat, but imparts sufficient energy to the tissue for incisions and cauterization when necessary.

The control system in the *Thomas* patent is of the analog type. A phase lock loop (that includes a voltage controlled oscillator, a frequency divider, a power switch, a matching network and a phase detector), stabilizes the frequency applied to the hand piece. A microprocessor controls the amount of power by sampling the frequency, current and voltage applied to the hand piece, because these parameters change with load on the blade.

The power versus load curve in a generator in a typical ultrasonic surgical system, such as that described in the *Thomas* patent, has two segments. The first segment has a positive slope of increasing power as the load increases, which indicates constant current delivery. The second segment has a negative slope of decreasing power as the load increases, which indicates a constant or saturated output voltage. The regulated current for the first segment is fixed by the design of the electronic components and the second segment voltage is limited by the maximum output voltage of the design. This arrangement is inflexible since the power versus load characteristics of the output of such a system can not be optimized to various types of hand piece transducers and ultrasonic blades. The performance of traditional analog ultrasonic power systems for surgical instruments is affected by the component tolerances and their variability in the generator electronics due to changes in operating temperature. In particular, temperature changes can cause wide variations in key system parameters such as frequency lock range, drive signal level, and other system performance measures.

In order to operate an ultrasonic surgical system in an efficient manner, during startup the frequency of the signal supplied to the hand piece transducer is swept over a range to locate the resonance frequency. Once it is found, the generator phase lock loop locks on to the resonance frequency, continues to monitor the transducer current to voltage phase angle, and maintains the transducer resonating by driving it at the resonance frequency. A key function of such systems is to maintain the transducer resonating across load and temperature changes that vary the resonance frequency. However, these traditional ultrasonic drive systems have little to no flexibility with regards to adaptive frequency control. Such flexibility is key to the system's ability to discriminate undesired resonances. In particular, these systems can only search for resonance in one direction, i.e., with increasing or decreasing frequencies and their search pattern is fixed. The system cannot: (i) hop over other resonance modes or make any heuristic decisions, such as what resonance to skip or lock onto, and (ii) ensure delivery of power only when appropriate frequency lock is achieved.

The prior art ultrasonic generator systems also have little flexibility with regard to amplitude control, which would allow the system to employ adaptive control algorithms and decision making. For example, these fixed systems lack the ability to make heuristic decisions with regards to the output drive, e.g., current or frequency, based on the load on the blade and/or the current to voltage phase angle. It also limits the system's ability to set optimal transducer drive signal levels for consistent efficient performance, which would increase the useful life of the transducer and ensure safe operating conditions for the blade. Further, the lack of control over amplitude and frequency control reduces the system's abilty to perform diagnostic tests on the transducer/blade system and to support troubleshooting in general.

Some limited diagnostic tests performed in the past involve sending a signal to the transducer to cause the blade to move and the system to be brought into resonance or some other vibration mode. The response of the blade is then determined by measuring the electrical signal supplied to the transducer when the system is in one of these modes. The ultrasonic system described in EP-A-1199047 possesses the ability to sweep the output drive frequency, monitor the frequency response of the ultrasonic transducer and blade, extract parameters from this response, and use these parameters for system diagnostics. This frequency sweep and response measurement mode is achieved via a digital code such that the output drive frequency can be stepped with high resolution, accuracy, and repeatability not existent in prior art ultrasonic systems.

A problem associated with the prior art ultrasonic systems is blade breakage or cracking at points of high stress on the blade. Breakage and cracking of blades are two major causes of the ultrasonic generator failing to acquire lock or failing to maintain longitudinal displacement. For example, as the crack develops both the frequency of oscillation and the magnitude of mechanical impedance change to such an extent that the ultrasonic generator can no longer locate the resonance of the hand piece/blade. A more advanced generator may be able to lock onto a transducer coupled to such a blade. However, a cracked blade has a reduced ability to oscillate in the longitudinal direction. In this situation, an increased ability to locate the desired resonance upon which to lock is not useful, and may actually mask the loss of optimal cutting conditions.

Further, burdened or gunked blades, i.e., blades with dried blood, skin, hair and desiccated tissue built up around the blade at the point where the sheath surrounds the blade, present a greater load than clean blades. In particular, the gunk results in a load on the blade, and represents an increase in the mechanical impedance of the transducer presented to the ultrasonic generator.

This phenomenon has the following unwanted consequence. Ultrasonic generators possess a maximum operating voltage beyond which optimal operation of the hand piece/blade is lost. Many ultrasonic drivers attempt to maintain a constant drive current level to the transducer to keep the displacement at the blade tip constant in the presence of varying loads on the blade. As the impedance of the transducer is increased (as a result of tissue pressure, gunked tissue, etc.), the drive voltage must be increased to maintain the drive current at a constant level. Eventually, the loading of the blade becomes great enough such that the voltage reaches a maximum level, and any further loading of the blade results in a reduction of the drive current signal level.

As the current level of the drive signal is reduced, the displacement will begin to fall. The generator can drive an increasing load only as long as the hand piece/blade is not loaded such that the resonance point becomes unrecognizable (due to degradation of the signal to noise ratio or an inability of the hand piece/blade to resonate). As a consequence, the tissue applied force at maximum power, the maximum tissue applied force before losing the resonance signal, and the cutting/coagulating ability of the blade between these two operating points, become degraded.

In addition to the problems associated with loads on the blade, there is a buildup of heat at the coagulum. This buildup absorbs energy from the blade, and heats both the blade and sheath at this location. A cracked or broken blade loses the ability to resonant as well as a blade which is in good condition, and thus should be discarded. However, a gunked blade can be cleaned or used, and resonates as well as a new blade. In an operating room, access to either cracked and gunked blades for visual inspection is not practical. However, it is advantageous to differentiate between broken blades and those which are gunked, but otherwise in good condition, because a user can quickly and with confidence decide whether to discard or to clean an expensive blade. Cleaning a blade which is gunked verses discarding what is otherwise a good blade results in a substantial reduction in purchasing costs which are passed on to hospital patients as a savings.

Detection of debris on the blade, and the determination of the condition of tissue that the blade is in contact with are additional problems associated with conventional ultrasonic systems. Some ultrasonic blades are equipped with a sheath which covers the blade. The majority of the sheath is not in contact with the blade. Space (voids) between the sheath and the blade permits the blade to move freely. During use, this space can become filled with debris such as blood and tissue. This debris has a tendency to fill the space between the sheath and blade, and increase mechanical coupling between the blade and the sheath. As a result, undesired loading of the blade may increase, the temperature of the blade sheath may increase and the energy delivered to the tip may be reduced. In addition, if the debris sufficiently coagulates/hardens inside the sheath, the ability of the generator to initiate blade vibration while in contact with tissue may be prohibited. Moreover, vibration/start up of the blade in free air may also be inhibited.

### SUMMARY OF THE INVENTION

The invention is a method for distinguishing between gunked and cracked ultrasonically tuned blades in an ultrasonic surgical system; comprising the steps of:
applying a drive signal having a drive current level and a drive voltage level to an ultrasonic hand piece/blade using an ultrasonic generator across a predetermined frequency range, wherein said drive signal is applied at a first excitation level and at a second excitation level which is higher than said first excitation level;
for each of said excitation levels, measuring (a) the minimum impedance magnitude across said frequency range, or (b) the maximum phase difference between the drive current and the drive voltage across said frequency range;
displaying a first message on the liquid crystal display, if the minimum impedance magnitude obtained at said first excitation level is less than the minimum impedance magnitude obtained at said second excitation level, or the maximum phase difference obtained at said first excitation level is greater than the maximum phase difference obtained at said second excitation level; and
otherwise displaying a second message on the liquid crystal display.

In accordance with the invention, the method is performed irrespective of the age of the hand piece/blade, the temperature or specific type of hand piece or blade, and is not affected by self healing effects of slightly cracked blades. Moreover, in a preferred embodiment, the method facilitates the quantifiable determination of the amount of gunk on the blade. Absolute impedance measurements of the transducer or blade are unnecessary. Instead, only relative impedance measurements are required, which greatly simplifies the measuring criteria. In one embodiment, the method is used to evaluate the measured impedance differences when a system is first excited with a low displacement signal and then with a high displacement signal. This provides a way to measure the amount of gunk accumulation, and thereby a way to calculate/estimate the amount of heat generated at the sheath, as well as a way to calculate/estimate the amounts of degradation to the load curve of the ultrasonic system.

In an embodiment of the invention, a blade which possesses a lower resonance frequency at a predetermined drive voltage level is used to detect broken blades. The following procedure typically jiggles the blade, i.e., causes the blade to move quickly back and forth. First, the impedance and/or phase of the signal to the hand piece is measured at normal excitation levels over a range of frequencies about the resonance frequency. Second, the same measurements are made at a lower excitation (current) level. The measurements at the same frequencies for the normal and low level excitation of the blade are compared. The first or normal level measurements will change relative to the second or low level measurements, as the jiggled blade becomes more or less homogeneous at the low level. These high-low measurements, i.e., this jiggling, is repeated many times, and the amount of change in impedance is used to determine whether the blade is cracked. When using an unbroken blade, the impedance does not significantly change between such jiggling of the blade. However, if the blade is broken the jiggling will result in a change in the measurement because at the high level the blade partly separates, and at the low level the self heeling causes the impedance pattern to change. detected, even though the dampening has substantially changed. For example, if the blade is not used for an extended period of time, blood/tissue which enters the sheath gap during use will coagulate and substantially dampen the blade. This change in dampening can be observed and detected, and the user can be alerted to the change.

In an additional embodiment of the invention, the condition of tissue is determined. A blade in contact with tissue possesses a dampened response which is relative to the condition of the tissue and the pressure applied. For a given blade, tissue condition and contact pressure, the amount of dampening changes as the tissue condition changes. Consequently, the condition of tissue is determined by obtaining relative measurements of dampening while the blade is in contact with the tissue. This is accomplished by periodically interrupting the normal drive signal to the transducer, providing a test drive signal to obtain a brief dampening measurement, and then reapplying the normal drive signal to the transducer. This does not degrade the overall performance of the ultrasonic system, and does not interrupt the continuous use of the system.

The method can be advantageously used to focus on a single event, such as the coagulation of a vessel. When the user of the ultrasonic system begins to coagulate the blood vessel, the console measures the initial dampening level and periodically continues dampening measurements until the dampening level has adequately changed and/or the rate of dampening has appropriately changed. When the appropriate dampening response is reached (e.g., when tissue of one type or condition has been severed and the blade has encountered tissue of another type or condition), the console indicates the status to the user or stops/reduces energy delivery to the hand piece/blade. The energy delivery is adjustable in real-time according to the measured on-going dampening levels. The dampening level is displayable for consideration by the user, or is usable in an algorithm to control energy delivery to the hand piece/blade.

It is also desirable to know the relative condition of skin tissue, especially the condition of the tissue which has been altered by ultrasonic energy. Assessing the condition of tissue permits the proper adjustment of the energy applied to the tissue, and also permits the indication of when adequate cauterization, dessication, or other tissue effects have occurred. Together, these provide a means to determine whether additional energy or whether an extension of the application time of the energy is required. Further, the assessment of the tissue condition permits the avoidance

### BRIEF DESCRIPTION OF THE DRAWINGS

The foregoing and other advantages ahd features of the invention will become more apparent from the detailed description of the preferred embodiments of the invention given below with reference to the accompanying drawings in which:
FIG. 1 is an illustration of impedance vs frequency plots for an ultrasonic blade which is cracked, gunked or good when driven at a low signal level or a high signal level;
FIG. 2 is an illustration of phase vs frequency plots for an ultrasonic blade which is cracked, gunked or good when driven at a low signal level or a high signal level;
FIG. 3 is an illustration of impedance vs frequency plots for an ultrasonic blade which is cracked or has completely broken away from a hand piece when driven at a low signal level or a high signal level;
FIG. 4 is an illustration of a console for an ultrasonic surgical cutting and hemostasis system, as well as a hand piece and foot switch in which the method of the present invention is implemented;
FIG. 5 is a schematic view of a cross section through the ultrasonic scalpel hand piece of the system of FIG. 4;
FIGS. 6(a) and 6(b) are block diagrams illustrating an ultrasonic generator for implementing the method of the present invention;
FIGS. 7(a) and (b) are flow charts illustrating a preferred embodiment of the method of the invention;
FIGS. 8(a) and 8(b) are flow charts illustrating an alternative embodiment of the invention;

### DETAILED DESCRIPTION OF THE PREFERRED EMBODIMENTS

Impedance measurements of mechanical or acoustic systems obtained at high excitation levels provides much more information than impedance measurements obtained at low excitation levels. Moreover, comparisons of impedance measurements between low and high energy excitation levels provide even more detailed information about the condition of the hand piece/blade. The condition of the hand piece/blade falls into three main categories.

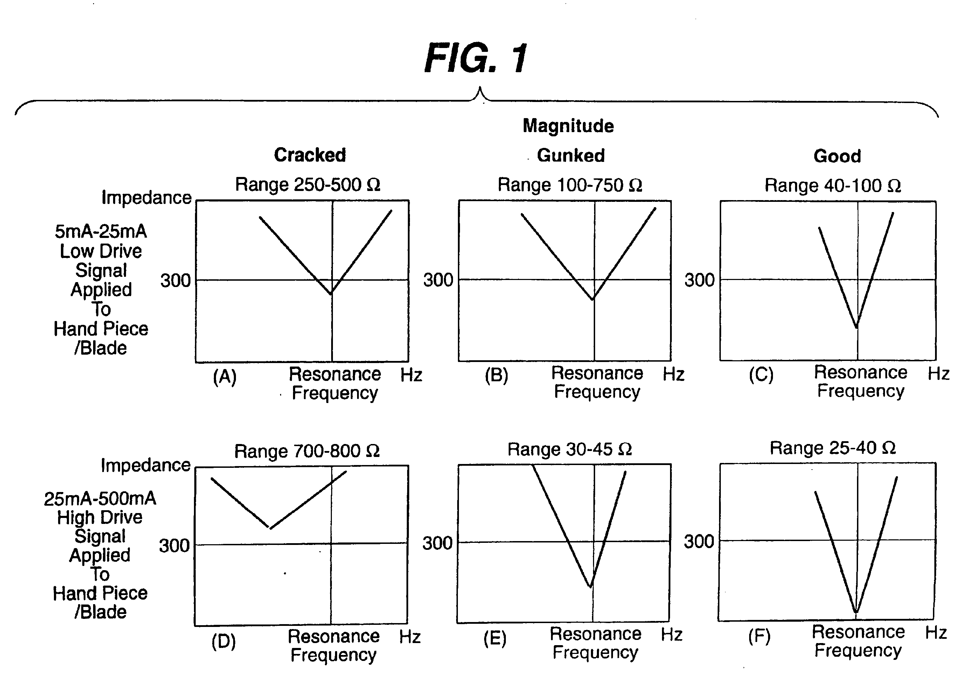

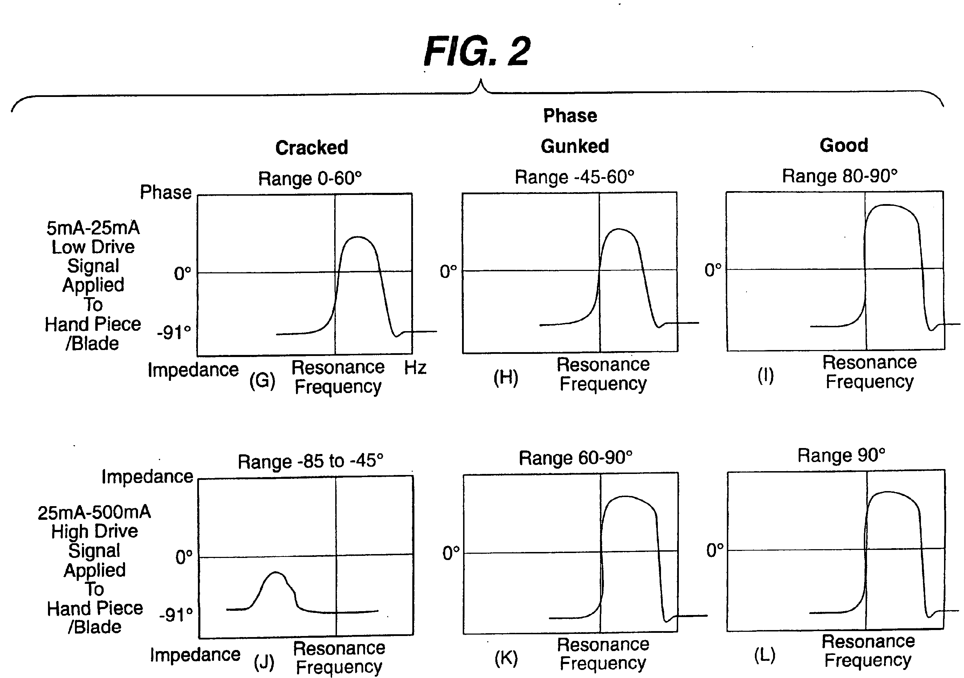

Firstly, gunked blades and new clean blades belong to the same category because silicon anti-node supporters and other mechanical inefficiencies, such as mechanical resistance in the longitudinal direction of the blade, have the same dampening effect as gunk upon the hand piece/blade. In particular, clean/gunked systems become much better resonators as the excitation amplitude is increased, that is they become higher Q systems (the minimum impedance gets markedly lower and the maximum phases get markedly higher; see FIG. 1 and compare the impedance vs. frequency plot shown in B to the impedance *vs*. frequency plot shown in E, and see FIG. 2 and compare the phase vs. frequency plot shown in H to the phase vs. frequency plot shown in K). The degree of improvement is relative to the loading effect of the gunk involved. As the excitation level changes, there is a minimal change in the resonance frequency which is close to the resonance frequency of a clean hand piece/blade. At a low excitation level, such as 5mA, a cracking or slightly cracked blade is generally self healing and looks very much like a gunked blade (see FIG. 1 and compare the impedance *vs*. frequency plot shown in A to the impedance *vs*. frequency plot shown in B, and see FIG. 2 and compare the phase vs. frequency plot shown in G to the phase vs. frequency plot shown in H). The self healing characteristic, in which at a molecular level the blade becomes more homogeneous if not overly excited, results in an optimally tuned system. At low excitation levels, the surfaces at the interface of the crack do not behave like disjoint surfaces, and are held in close contact to each other by the parts of the blades which are still intact. In this situation, the system appears "healthy."

Secondly, at larger excitation levels, such as 25mA or greater, stresses at the crack become large enough such that the portion of the blade which is distal to the crack no longer acts as if it is intimately connected to the proximal portion of the blade. A characteristic of these hand piece/blades is their non-linear behavior (i.e., very sharp non-continuous changes in impedance magnitudes and phase) which occur as the resonance frequency is approached and the stresses in the shaft of the hand piece become large. As the frequency approaches resonance of the "intact blade", the stresses become increasingly greater until at a certain point the blade suddenly becomes disjointed at the crack. This effectively shortens the blade, and the resonator or blade will possess completely different resonance impedance characteristics. Typically, the impedance of such a shorter blade results in a hand piece/blade which possesses a lower Q, as well as a lower frequency of resonance (see FIG. 1 and compare the respective impedance vs. frequency plots shown in A and C to the respective impedance vs. frequency plots shown in D and F, and see FIG. 2 and compare the respective phase vs. frequency plots shown in G and I to the respective phase vs. frequency plots shown in J and L).

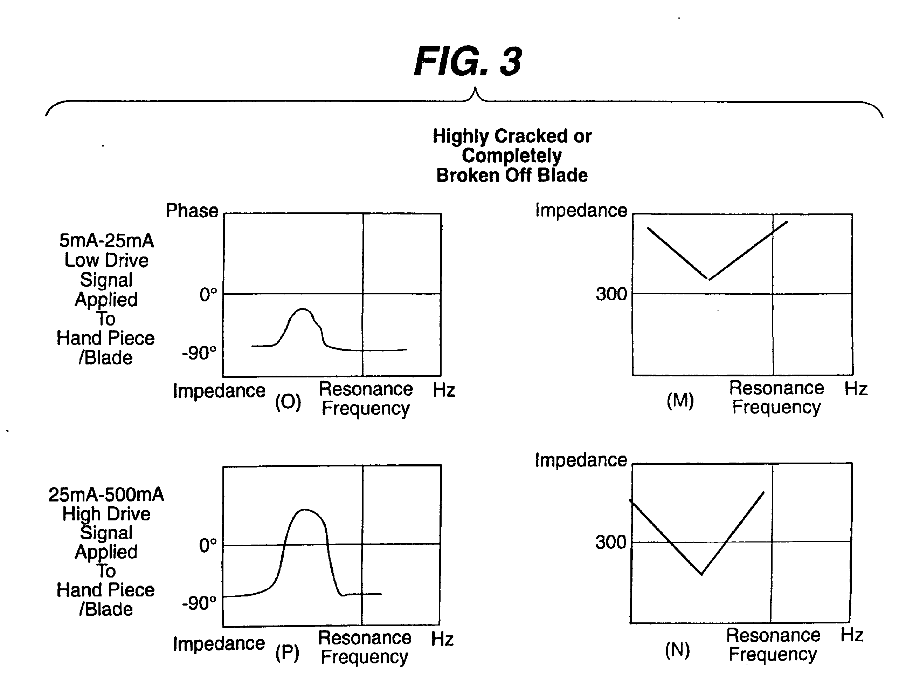

Lastly, severely cracked blades include, but are not limited to, blades having tips which have completely fallen off due to mechanical stress acting on the blades. These blades are substantially equivalent to gunked blades. However, they are not useful for cutting/coagulating tissue in longitudinal directions. Such blades appear to behave similarly in that they present improved (if only marginally) impedance characteristics at higher excitation levels, and their frequency of resonance is not affected by higher excitation levels. However, they can be differentiated from gunked blades due to their extremely high impedance level. This requires absolute measurements, but only coarse levels of precision are required. Generally, the resonance frequency of the transducer or blade is shifted far away from the normal resonance that is typically used for a specific ultrasonic system. This shift is usually a downward shift of the resonance frequency of about 2 kilohertz. When excited with a higher level of current and compared with a lower level of current, the impedance magnitude, resonance frequency and maximum phase at resonance are quantitatively far different than the corresponding characteristics of blades which are only gunked (see FIG. 3 and compare the impedance *vs*. frequency plot shown in M to the impedance *vs*. frequency plot shown in N, and compare the phase *vs*. frequency plot shown in O to the phase *vs*. frequency plot shown in P). In this case, the hand piece/blade typically possesses a magnitude of impedance at resonance which is approximately 400 ohms higher for cracked blades than that of heavily gunked but otherwise good blades. Of note, Figs. 1-3 show values that are exemplify a particular US system, and absolute values are dependent upon actual the actual design of the system.

Most broken or cracked blades have self healing characteristics associated with them. The self healing characteristic, in which at a molecular level the blade becomes more homogeneous if not overly excited, results in an optimally tuned system. This homogeneity is disturbed at a high excitation level, resulting in an untuned system. When cracked or broken blades are un-energized for an extended period of time, or if energized at a low intensity for a period of time, such blades present a mechanical impedance to the ultrasonic generator that is closer to the mechanical impedance which is exhibited by an unbroken blade. At high excitation levels, the portion of the blade distal to the crack is no longer intimately connected to the hand piece/blade. The effect of the high excitation level upon the blade is that the portion of the blade proximal to the crack "bangs" against the portion of the blade distal to the crack, which causes a loading effect which is greater than the loading effect at low excitation displacement levels.

In other words, in the frequency range of approximately 1,000 Hz, centered around the resonance frequency of an unbroken blade, the same type of broken blade will exhibit one impedance sweep characteristic at a low voltage excitation of the drive transducer and another at a high voltage excitation level. In contrast, an unbroken blade exhibits the same impedance at both excitation levels, as long as the impedance measurement is performed quickly enough, or at a low enough displacement level such that the transducer or the blade does not overheat. Heat causes the resonance point to shift downwards in frequency. This heating effect is most prevalent when the magnitude of the excitation frequency approaches the resonance frequency due to gunk.

In addition, an excitation threshold exists, below which the blade "self heals" and presents increasingly "tuned" impedance levels (over time) to the driving elements, and above which the crack presents a discontinuity to the homogeneity of the blade. Thus, below this threshold, the impedance characteristic may exhibit the same characteristic for all excitation levels. The blade may also appear to be healing itself at these lowered excitation levels. Above this excitation threshold, the impedance may possess a different appearance than the low impedance measurements, but may still not change with increasing levels of excitation. This excitation threshold is different for each type of blade as well as each cracked location on the blade, and is modulated by the amount of gunk loading the distal part of the blade.

Some of the impedance differences seen in a system containing a broken blade (which are not seen in a system containing an unbroken blade), when first driven with a low excitation current and then with a high excitation current, are a lower Q (i.e., a lower minimum impedance) over a frequency span centered about the resonance frequency of an unbroken blade, i.e., a higher minimum impedance and/or a lower maximum impedance. It could also mean a higher "phase margin", i.e., Fa-Fr (where Fa-Fr is anti-resonance frequency minus the resonance frequency respectively). Other differences are a higher impedance at a frequency slightly above the anti-resonance frequency of the normally operating system, a higher impedance at a frequency slightly below the resonance point of a properly working system, or a large change in the resonance frequency. Gunked or loaded blades connected to a drive system exhibit somewhat opposite effects to that of a cracked blade. A system loaded in this manner exhibits an increasingly improved Q around the resonance point as the excitation voltage is increased.

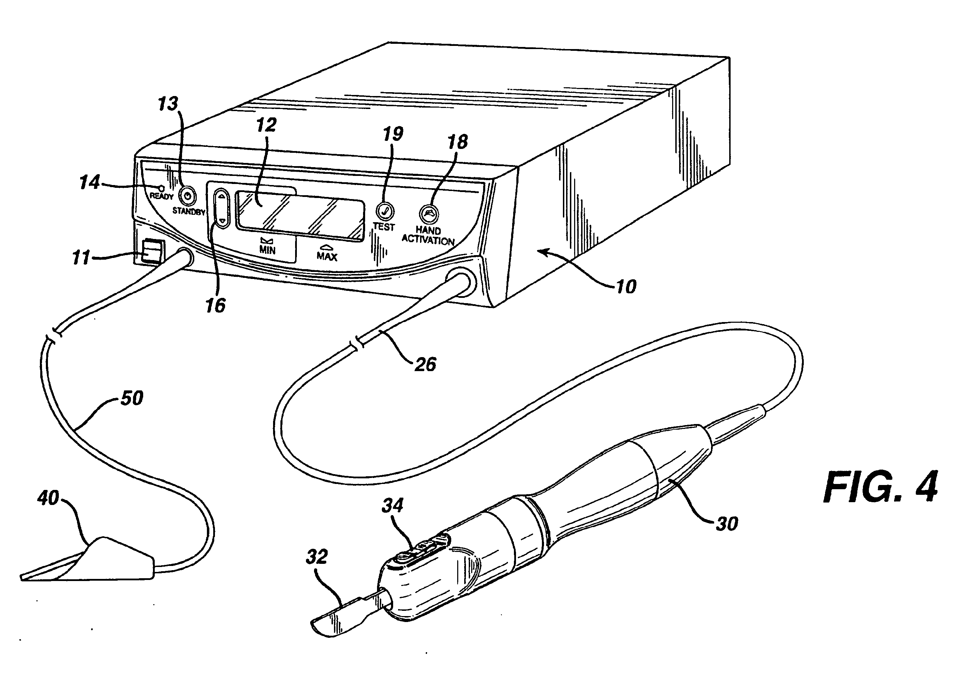

FIG. 4 is an illustration of a system for implementing the method in accordance with the invention. By means of a first set of wires in cable 20, electrical energy, i.e., drive current, is sent from the console 10 to a hand piece 30 where it imparts ultrasonic longitudinal movement to a surgical device, such as a sharp scalpel blade 32. This blade can be used for simultaneous dissection and cauterization of tissue. The supply of ultrasonic current to the hand piece 30 may be under the control of a switch 34 located on the hand piece, which is connected to the generator in console 10 via wires in cable 20. The generator may also be controlled by a foot switch 40, which is connected to the console 10 by another cable 50. Thus, in use a surgeon may apply an ultrasonic electrical signal to the hand piece, causing the blade to vibrate longitudinally at an ultrasonic frequency, by operating the switch 34 on the hand piece with his finger, or by operating the foot switch 40 with his foot.

The generator console 10 includes a liquid crystal display device 12, which can be used for indicating the selected cutting power level in various means such, as percentage of maximum cutting power or numerical power levels associated with cutting power. The liquid crystal display device 12 can also be utilized to display other parameters of the system. Power switch 11 I is used to turn on the unit. While it is warming up, the "standby" light 13 is illuminated. When it is ready for operation, the "ready" indicator 14 is illuminated and the standby light goes out. If the unit is to supply maximum power, the MAX button 15 is depressed. If a lesser power is desired, the MIN button 17 is activated. The level of power when MIN is active is set by button 16.

When power is applied to the ultrasonic hand piece by operation of either switch 34 or 40, the assembly will cause the surgical scalpel or blade to vibrate longitudinally at approximately 55.5 kHz, and the amount of longitudinal movement will vary proportionately with the amount of driving power (current) applied, as adjustably selected by the user. When relatively high cutting power is applied, the blade is designed to move longitudinally in the range of about 40 to 100 microns at the ultrasonic vibrational rate. Such ultrasonic vibration of the blade will generate heat as the blade contacts tissue, i.e., the acceleration of the blade through the tissue converts the mechanical energy of the moving blade to thermal energy in a very narrow and localized area. This localized heat creates a narrow zone of coagulation, which will reduce or eliminate bleeding in small vessels, such as those less than one millimeter in diameter. The cutting efficiency of the blade, as well as the degree of hemostasis, will vary with the level of driving power applied, the cutting rate of the surgeon, the nature of the tissue type and the vascularity of the tissue.

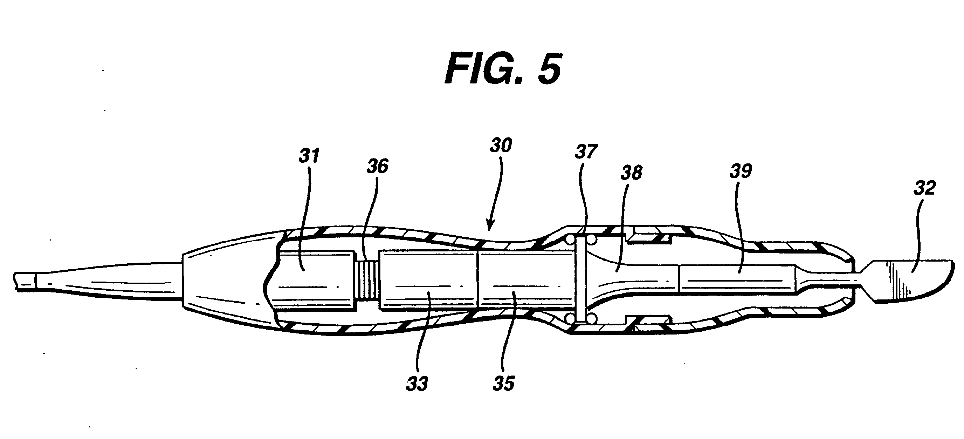

As illustrated in more detail in FIG. 5, the ultrasonic hand piece 30 houses a piezoelectric transducer 36 for converting electrical energy to mechanical energy that results in longitudinal vibrational motion of the ends of the transducer. The transducer 36 is in the form of a stack of ceramic piezoelectric elements with a motion null point located at some point along the stack. The transducer stack is mounted between two cylinders 31 and 33. In addition a cylinder 35 is attached to cylinder 33, which in turn is mounted to the housing at another motion null point 37. A horn 38 is also attached to the null point on one side and to a coupler 39 on the other side. Blade 32 is fixed to the coupler 39. As a result, the blade 32 will vibrate in the longitudinal direction at an ultrasonic frequency rate with the transducer 36. The ends of the transducer achieve maximum motion with a portion of the stack constituting a motionless node, when the transducer is driven with a current of about 380mA RMS at the transducers' resonant frequency. However, the current providing the maximum motion will vary with each hand piece and is a valve stored in the nonvolatile memory of the hand piece so the system can use it.

The parts of the hand piece are designed such that the combination will oscillate at the same resonant frequency. In particular, the elements are tuned such that the resulting length of each such element is one-half wavelength. Longitudinal back and forth motion is amplified as the diameter closer to the blade 32 of the acoustical mounting horn 38 decreases. Thus, the horn 38 as well as the blade/coupler are shaped and dimensioned so as to amplify blade motion and provide harmonic vibration in resonance with the rest of the acoustic system, which produces the maximum back and forth motion of the end of the acoustical mounting horn 38 close to the blade 32. A motion at the transducer stack is amplified by the horn 38 into a movement of about 20 to 25 microns. A motion at the coupler 39 is amplified by the blade 32 into a blade movement of about 40 to 100 microns.

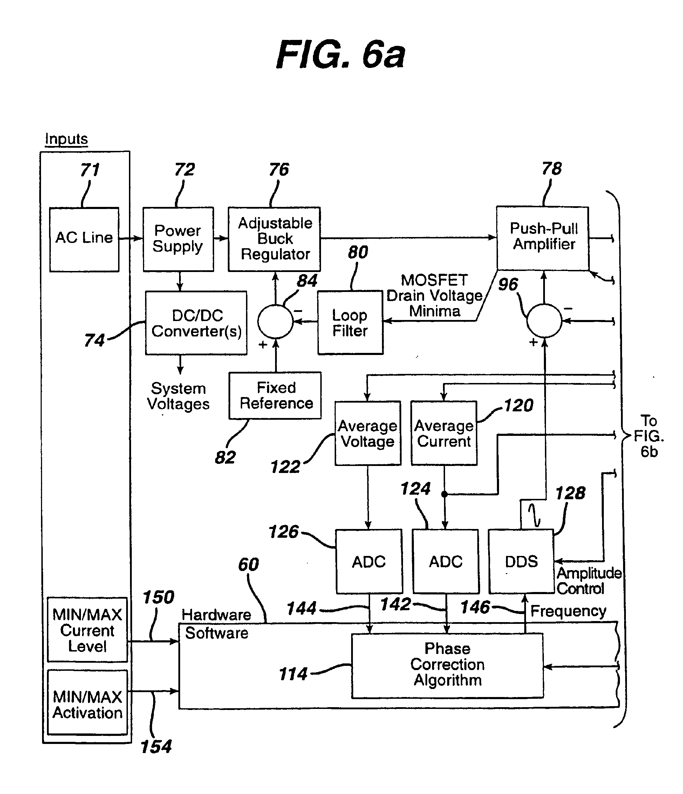

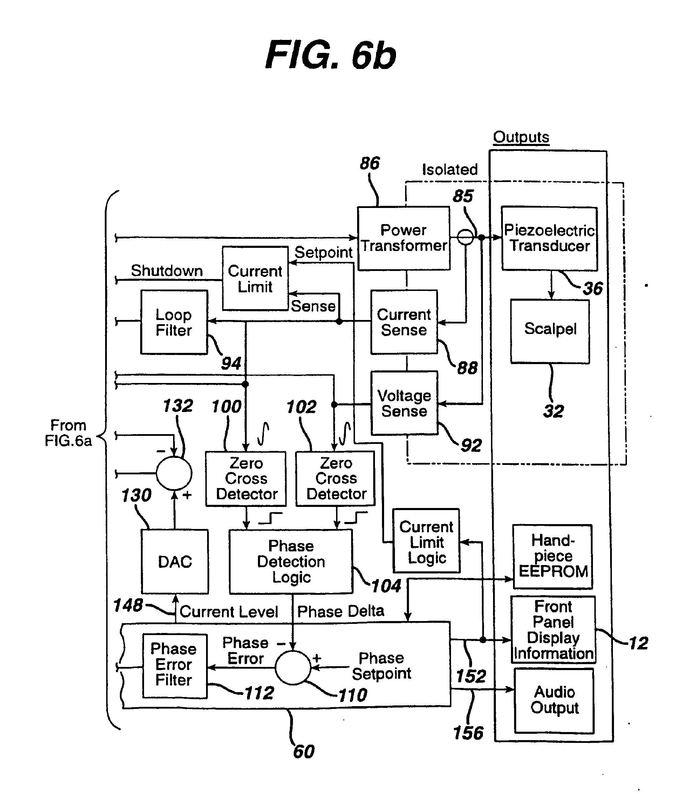

The system which creates the ultrasonic electrical signal for driving the transducer in the hand piece is illustrated in FIGS. 6(a) and 6(b). This drive system is flexible and can create a drive signal at a desired frequency and power level setting. A DSP 60 or microprocessor in the system is used for monitoring the appropriate power parameters and vibratory frequency as well as causing the appropriate power level to be provided in either the cutting or coagulation operating modes. The DSP 60 or microprocessor also stores computer programs which are used to perform diagnostic tests on components of the system, such as the hand piece/blade.

For example, under the control of a program stored in the DSP or microprocessor 60, such as a phase correction algorithm, the frequency during startup can be set to a particular value, e.g., 50 kHz. It can than be caused to sweep up at a particular rate until a change in impedance, indicating the approach to resonance, is detected. Then the sweep rate can be reduced so that the system does not overshoot the resonance frequency, e.g., 55 kHz. The sweep rate can be achieved by having the frequency change in increments, e.g., 50 cycles. If a slower rate is desired, the program can decrease the increment, e.g., to 25 cycles which both can be based adaptively on the measured transducer impedance magnitude and phase. Of course, a faster rate can be achieved by increasing the size of the increment. Further, the rate of sweep can be changed by changing the rate at which the frequency increment is updated.

If it is known that there is a undesired resonant mode, e.g., at say 51 kHz, the program can cause the frequency to sweep down, e.g., from 60 kHz, to find resonance. Also, the system can sweep up from 50 kHz and hop over 51 kHz where the undesired resonance is located. In any event, the system has a great degree of flexibility

In operation, the user sets a particular power level to be used with the surgical instrument. This is done with power level selection switch 16 on the front panel of the console. The switch generates signals 150 that are applied to the DSP 60. The DSP 60 then displays the selected power level by sending a signal on line 152 (FIG. 6(b)) to the console front panel display 12. Further, the DSP or microprocessor 60 generates a digital current level signal 148 that is converted to an analog signal by digital-to-analog converter (DAC) 130.

To actually cause the surgical blade to vibrate, the user activates the foot switch 40 or the hand piece switch 34. This activation puts a signal on line 154 in FIGS. 6(a). This signal is effective to cause power to be delivered from push-pull amplifier 78 to the transducer 36. When the DSP or microprocessor 60 has achieved lock on the hand piece transducer resonance frequency and power has been successfully applied to the hand piece transducer, an audio drive signal is put on line 156. This causes an audio indication in the system to sound, which communicates to the user that power is being delivered to the hand piece and that the scalpel is active and operational.

In order to obtain the impedance measurements and phase measurements, the DSP 60 and the other circuit elements of FIGS. 6(a) and 6(b) are used. In particular, push-pull amplifier 78 delivers the ultrasonic signal to a power transformer 86, which in turn delivers the signal over a line 85 in cable 26 to the piezoelectric transducers 36 in the hand piece. The current in line 85 and the voltage on that line are detected by current sense circuit 88 and voltage sense circuit 92. The current and voltage sense signals are sent to average voltage circuit 122 and average current circuit 120, respectively, which take the average values of these signals. The average voltage is converted by analog-to-digital converter (ADC) 126 into a digital code that is input to DSP 60. Likewise, the current average signal is converted by analog-to-digital converter (ADC) 124 into a digital code that is input to DSP 60. In the DSP the ratio of voltage to current is calculated on an ongoing basis to give the present impedance values as the frequency is changed. A significant change in impedance occurs as resonance is approached.

The signals from current sense 88 and voltage sense 92 are also applied to respective zero crossing detectors 100, 102. These produce a pulse whenever the respective signals cross zero. The pulse from detector 100 is applied to phase detection logic 104, which can include a counter that is started by that signal. The pulse from detector 102 is likewise applied to logic circuit 104 and can be Used to stop the counter. As a result, the count which is reached by the counter is a digital code on line 104, which represents the difference in phase between the current and voltage. The size of this phase difference is also an indication of resonance. These signals can be used as part of a phase lock loop that cause the generator frequency to lock onto resonance, e.g., by comparing the phase delta to a phase set point in the DSP in order to generate a frequency signal to a direct digital synthesis (DDS) circuit **128** that drives the push-pull amplifier **78**.

Further, the impedance and phase values can be used as indicated above in a diagnosis phase of operation to detect if the blade is loose. In such a case the DSP does not seek to establish phase lock at resonance, but rather drives the hand piece at particular frequencies and measures the impedance and phase to determine if the blade is tight.

Since the DSP has measured and stored values of impedance and phase at particular frequencies and excitation levels, it can plot responses such as those in FIGS. 1-3. Thus, it can calculate the Q of the hand piece as well.

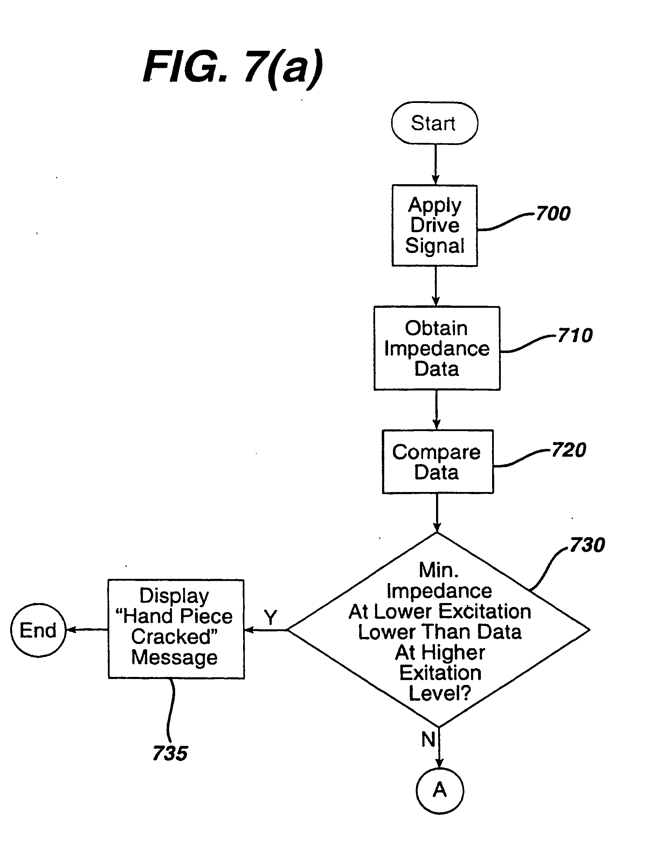

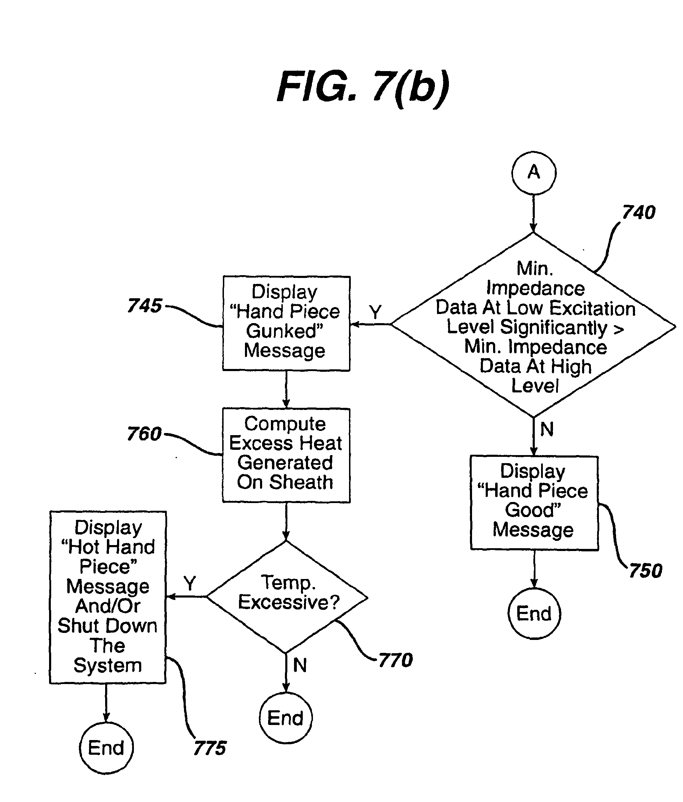

FIGS. 7(a) and 7(b) are flow charts illustrating a preferred embodiment of the invention. Under control of the program stored in the DSP or microprocessor 60 shown in FIGS. 6(a) and 6(b), the method of the invention is implemented by using the ultrasonic driver unit to excite the hand piece/blade and obtain impedance data over a frequency range of 50 to 60 kilohertz, as indicated in step **700**. Magnitude of impedance and phase of impedance data is obtained for two or more excitation levels ranging from a first current level to second current level, such as from 5mA to 50mA, as indicated in step **710**. Data within this range is collected in any order, including sweeping up or down in a discontinuous sampling sequence: To identify or discriminate between gunked and cracked blades, comparisons are performed between characteristics measurements, namely the magnitude of the lowest impedance obtained or the maximum phase between the current and the voltage.

If the impedance data sweep(s) at a lower excitation level reveal that the minimum impedance magnitude is lower than the minimum impedance magnitude obtained at a higher excitation level (step **730**), then the blade or the hand piece is cracked, and a "Blade Cracked" message is displayed on the LCD 12, as indicated in step **735**. Alternatively, whether the difference between the frequency of resonance at a high level and the frequency of resonance at a low levet is less than or equal to a threshold, such as 20 Hz, can be used to indicated whether a cracked blade exists. If, on the other hand, the lower excitation sweep(s) show little or no change in resonance frequency or a higher minimum impedance than the higher excitation sweeps (step 740), then the blade or hand piece is gunked, and a "Gunked Blade" message is displayed on the LCD 12, as indicated in step **745**. Further, the amount of gunking is determined by the differences in the impedance magnitudes which are obtained, and communicated to the user during display of the "Blade Gunked" message. The amount of excess heat generation on the sheath at the location of the gunk is computed, as indicated in step **760**. Excess heat may be estimated by calculating the relative difference in magnitude of the impedance measurements. If the temperature build up of heat will be excessive, a "Hot Blade" warning message is displayed on the LCD 12 and/or the user is instructed to shut down the system, as indicated in step **775**. If, on the other, hand, the heat Will not be excessive, the diagnostic test is terminated. Of note, the hot blade warning message is dependant on the blade characteristics. Heat generated within a particular blade design may be determined by using an I²R power-to-heat conversion for a given blade. It should be noted that the all of described measurements procedures may be performed using the DSP or microprocessor 60 in the ultrasonic generator. However, other devices may also be used to perform the measurements, such as a CPU, a Programmable Logic Device (PLD), or the like.

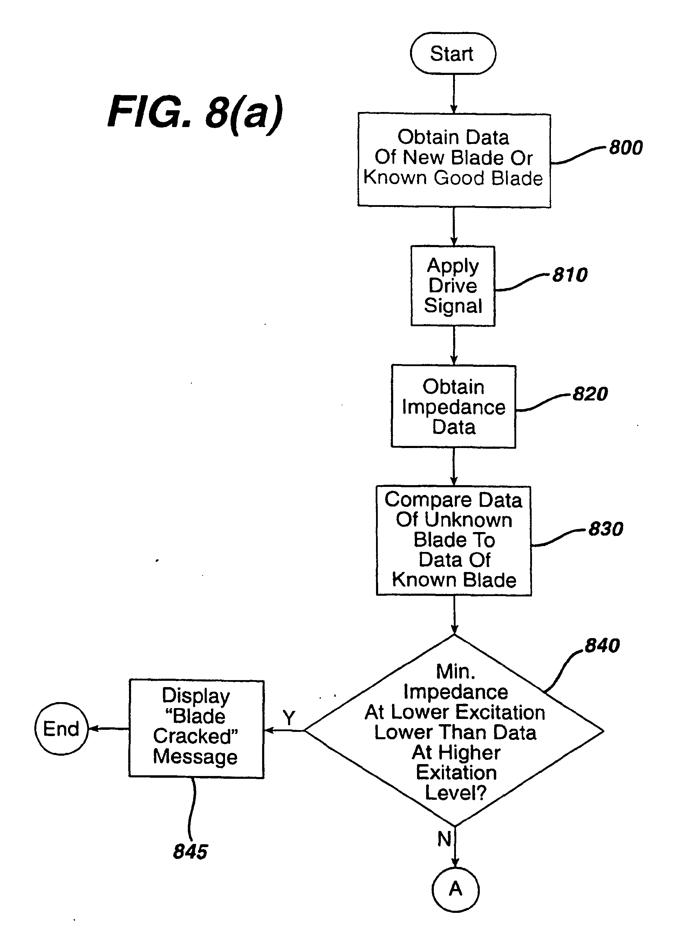

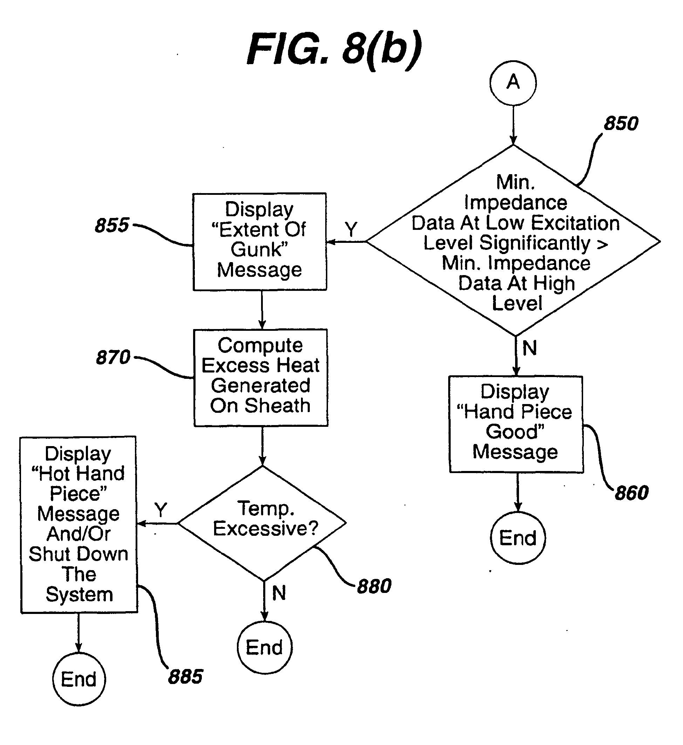

FIGS. 8(a) and 8(b) are flow charts illustrating an alternative embodiment of the invention. To increase the accuracy of the measurements, measurements of data from an initial test of a known good blade is compared to measurement data of a blade in an unknown condition. A threshold based on defined boundaries or ratios to a known good blade characteristics is calculated: ' As a result, testing accuracy is increased and less pronounced mal-effects on blades are detected. In addition, the ability to distinctly determine the extent of gunking is also provided. This is due to the attainment and use of a greater level of blade-specific measurement data for comparison, rather than the use of expected behavior data associated with generic good blades.

In an embodiment, instead of obtaining data by performing a test of the actual blade on the hand piece, the data can be obtained from a data source for the particular blade model which is in the blade ID or entered in the console, or the like. For details relating to blade ID, reference is made to EP-A-1260185.

The method permits the determination of whether the blade is in a severe condition or whether it is marginally problematic. In this case, the user can try to clean the blade and perform another test to measure the progress of cleaning and to help the user determine whether the cleaning of the blade is effective or ineffective. In embodiments, the "grading" may be used without the benefit of "known good blade" characteristics by providing a relative gunk score before and after cleaning to indicate how effectively the blade was cleaned. In alternative embodiments, the method is periodically initiated automatically by the console of the generator.

Under control of the program stored in the DSP or microprocessor 60 shown in FIGS. 6(a) and 6(b), the method of the invention is implemented by obtaining impedance data of a new blade or blade which is in good condition, as indicated in step **800**. The ultrasonic driver unit is used to excite- the hand piece/blade and obtain impedance data over a frequency range of 50 to 60 kilohertz, as indicated in step **810**. Magnitude of impedance and phase of impedance data is obtained for two or more excitation levels ranging from a first current level to second current level, such as from 5mA to 50mA, as indicated in step **820**, Data within this range is collected in any order, including sweeping up or down in a discontinuous sampling sequence. To identify or discriminate between gunked and cracked blades, comparisons are performed between characteristics measurements, namely the magnitude of the lowest impedance obtained or the maximum phase between the drive current and the drive voltage, the resonance frequency of the blade, and/or an evaluation of the non-linearity and/or continuousness of the measured data, as indicated in step **830**.

If the impedance data sweep(s) at a lower excitation level reveal that the minimum impedance magnitude is lower than the minimum impedance magnitude obtained at a higher excitation level (step **840**), then the blade or the hand piece is cracked, and a "Blade Cracked" message is displayed on the LCD 12, as indicated in step **845**. If, on the other hand, the lower excitation sweep(s) show a higher minimum impedance than the higher excitation sweeps (step **850**), then the blade or hand piece is gunked, and a "Extent of Gunk" message is displayed on the LCD 12; as indicated in step 855. Further, the amount of gunking is determined by the differences in the impedance magnitudes which are obtained, and communicated to the user during display of the "Extent of Gunk" message. The amount of excess heat generation on the sheath at the location of the gunk is computed, as indicated in step **870**. Excess heat may be estimated by calculating the relative difference in magnitude of the impedance measurements. If the temperature build up of heat will be excessive, a "Hot Blade" warning message is displayed on the LCD 12 and/or the user is instructed to shut down the system, as indicated in step **885**. If, on the other hand, the heat will not be excessive, the diagnostic test is terminated. As stated previously, the hot blade warning message is dependant on the blade characteristics. Heat generated within a particular blade design may be determined by using an I²R power-to-heat conversion for a given blade. In addition, the described measurement procedures may also be performed using the DSP or microprocessor 60 in the ultrasonic generator. However, other devices may also be used to perform the measurements, such as a CPU, Programmable Logic Device (PLD), or the like.

Using the method of the present invention, the state of a blade (i.e., whether the blade is cracked, gunked or good) during use in an operation room can be determined quickly, easily and accurately. The method(s) makes this determination independent of the type of hand piece/blade, the temperature of the hand piece/blade or the age of PZT, etc. The method also expedites the testing of unknown blades since less characteristic(s) data points are required to make conclusions due to the acquisition of blade-specific information. The invention informs a surgeon or nurse whether to discard a broken hand piece/blade, while also providing an opportunity to clean a gunked blade.

Although the invention has been described and illustrated in detail, it is to be clearly understood that the same is by way of illustration and example, and is not to be taken by, way of limitation. The scope of the present invention is to be limited only by the terms of the appended claims.

## Claims

1. A method for distinguishing between gunked and cracked ultrasonically tuned blades in an ultrasonic surgical system, comprising the steps of:
applying a drive signal having a drive current level and a drive voltage level to an ultrasonic hand piece/blade using an ultrasonic generator across a predetermined frequency range, wherein said drive signal is applied at a first excitation level and at a second excitation level which is higher than said first excitation level;
for each of said excitation levels, measuring. (a) the minimum impedance magnitude across said frequency range, or (b) the maximum phase difference between the drive current and the drive voltage across said frequency range;
displaying a first message on the liquid crystal display, if the minimum impedance magnitude obtained at said first excitation level is less than the minimum impedance magnitude obtained at said second excitation level, or the maximum phase difference obtained at said first excitation level is greater than the maximum phase difference obtained at said second excitation level; and
otherwise displaying a second message on the liquid crystal display.

2. The method claim 1, wherein the predetermined frequency range is from 50 kHz to 60 kHz.

3. The method of claim 1, wherein the step of displaying the first message comprises displaying a "Blade Cracked" message on the liquid crystal display.

4. The method of claim 1, wherein the first excitation level ranges from 5mA to 25mA.

5. The method of claim 1, wherein the second excitation level ranges from 25 mA to 500mA.

6. The method of any one of claims 1 to 5, wherein the step of displaying the second message comprises displaying a "Blade Gunked" or "Extent of Gunk" message on the liquid crystal display.

7. The method of claim 1, wherein said measuring step comprises measuring the minimum impedance magnitude across said frequency range, and said method further comprises the step of computing excess heat generated on a sheath of the hand piece/blade.

8. The method of claim 7, wherein said excess heat is computed by calculating differences between impedance magnitudes.

9. The method of claim 8, wherein the differences between impedance magnitudes are displayed during the step of displaying the second message.

10. The method of claim 7, further comprising the steps of:
at least one of displaying a third message on the liquid crystal display, if said excess heat indicates that the hand piece/blade is hot, and shutting down the ultrasonic surgical system.

11. The method of claim 10, wherein the step of displaying the third message comprises displaying a "Hot Hand Piece" message on the liquid crystal display.

## Patentansprüche

1. Verfahren zum Unterscheiden zwischen verschmutzten und gebrochenen Ultraschall betriebenen Klingen in einem chirurgischen Ultraschallsystem, umfassend die Schritte:
Anlegen eines Steuersignals mit einem Steuerstrompegel und einem Steuerspannungspegel an ein Ultraschallhandstück/eine Ultraschallklinge unter Verwendung eines Ultraschallgenerators über einen vorab festgelegten Frequenzbereich, wobei das Steuersignal auf einem ersten Anregungspegel und einem zweiten Anregungspegel angelegt wird, der höher als der erste Anregungspegel ist;
für jeden der Anregungspegel Messen (a) des minimalen Impedanzwerts über den Frequenzbereich oder (b) der maximalen Phasendifferenz zwischen dem Steuerstrom und der Steuerspannung über den Frequenzbereich;
Anzeigen einer ersten Meldung auf der Flüssigkristallanzeige, wenn der bei dem ersten Anregungspegel erhaltene minimale Impedanzwert geringer als der bei dem zweiten Anregungspegel erhaltene minimale Impedanzwert ist oder die bei dem ersten Anregungspegel erhaltene maximale Phasendifferenz größer als die bei dem zweiten Anregungspegel erhaltene maximale Phasendifferenz ist; und
anderenfalls Anzeigen einer zweiten Meldung auf der Flüssigkristallanzeige.

2. Verfahren nach Anspruch 1, **dadurch gekennzeichnet, daß** der vorab festgelegte Frequenzbereich von 50 kHz bis 60 kHz verläuft.

3. Verfahren nach Anspruch 1, **dadurch gekennzeichnet, daß** der Schritt des Anzeigens der ersten Meldung Anzeigen einer "Klinge gebrochen"-Meldung auf der Flüssigkristallanzeige umfaßt.

4. Verfahren nach Anspruch 1, **dadurch gekennzeichnet, daß** der erste Anregungspegel im Bereich von 5mA bis 25mA liegt.

5. Verfahren nach Anspruch 1, **dadurch gekennzeichnet, daß** der zweite Anregungspegel im Bereich von 25mA bis 500mA liegt.

6. Verfahren nach einem der Ansprüche 1 bis 5, **dadurch gekennzeichnet, daß** der Schritt des Anzeigens der zweiten Meldung Anzeigen einer "Klinge verschmutzt"- oder "Ausmaß der Verschmutzung"-Nachricht auf der Flüssigkristallanzeige umfaßt.

7. Verfahren nach Anspruch 1, **dadurch gekennzeichnet, daß** der Meßschritt Messen des minimalen Impedanzwerts über den Frequenzbereich umfaßt und daß das Verfahren ferner den Schritt des Berechnens von an einer Hülle des Handstücks / der Klinge erzeugter überschüssiger Wärme umfaßt.

8. Verfahren nach Anspruch 7, **dadurch gekennzeichnet, daß** die überschüssige Wärme durch Berechnen von Differenzen zwischen Impedanzwerten berechnet wird.

9. Verfahren nach Anspruch 8, **dadurch gekennzeichnet, daß** die Differenzen zwischen Impedanzwerten während des Schritts des Anzeigens der zweiten Meldung angezeigt werden.

10. Verfahren nach Anspruch 7, ferner umfassend die Schritte:
mindestens eins von Anzeigen einer dritten Meldung auf der Flüssigkristallanzeige, wenn die überschüssige Wärme anzeigt, daß das Handstück/die Klinge heiß ist, und Abschalten des chirurgischen Ultraschallsystems.

11. Verfahren nach Anspruch 10, **dadurch gekennzeichnet, daß** der Schritt des Anzeigens der dritten Meldung Anzeigen einer "Heißes Handstück"-Meldung auf der Flüssigkristallanzeige umfaßt.

## Revendications

1. Procédé de distinction entre des lames en polyester renforcé en fibres de verre et des lames cassées activées par des ultrasons dans un système chirurgical à ultrasons, comprenant les étapes consistant à :
appliquer un signal d'entraînement ayant un niveau de courant d'entraînement et un niveau de tension d'entraînement à une pièce/une lame à ultrasons en utilisant un générateur d'ultrasons au sein d'une plage de fréquences prédéterminée, où ledit signal d'entraînement est appliqué à un premier niveau d'excitation et à un second niveau d'excitation qui est supérieur audit premier niveau d'excitation ;
pour chacun desdits niveaux d'excitation, mesurer (a) la magnitude d'impédance minimum au sein de ladite plage de fréquences, ou (b) la différence de phase maximum entre le courant d'entraînement et la tension d'entraînement au sein de ladite plage de fréquences ;
afficher un premier message sur l'afficheur à cristaux liquides, si la magnitude d'impédance minimum obtenue audit premier niveau d'excitation est inférieure à la magnitude d'impédance minimum obtenue audit second niveau d'excitation, ou si la différence de phase maximum obtenue audit premier niveau d'excitation est supérieure à la différence de phase maximum obtenue audit second niveau d'excitation ; et
sinon, afficher un second message sur l'afficheur à cristaux liquides.

2. Procédé selon la revendication 1, dans lequel la plage de fréquences prédéterminée est de l'ordre de 50 kHz à 60 kHz.

3. Procédé selon la revendication 1, dans lequel l'étape d'affichage du premier message comprend l'affichage d'un message « lame cassée » sur l'afficheur à cristaux liquides.

4. Procédé selon la revendication 1, dans lequel le premier niveau d'excitation est de l'ordre de 5 mA à 25 mA.

5. Procédé selon la revendication 1, dans lequel le second niveau d'excitation est de l'ordre de 25 mA à 500 mA.

6. Procédé selon l'une quelconque des revendications 1 à 5, dans lequel l'étape d'affichage du second message comprend l'affichage d'un message « lame en polyester renforcé en fibres de verre » ou « polyester renforcé en fibres de verre » sur l'afficheur à cristaux liquides.

7. Procédé selon la revendication 1, dans lequel ladite étape de mesure comprend la mesure de la magnitude d'impédance minimum au sein de ladite plage de fréquences, et ledit procédé comprend en outre l'étape de calcul de l'excès de chaleur généré sur une gaine de la pièce/de la lame.

8. Procédé selon la revendication 7, dans lequel ledit excès de chaleur est calculé en calculant des différences entre les magnitudes d'impédance.

9. Procédé selon la revendication 8, dans lequel les différences entre les magnitudes d'impédance sont affichées pendant l'étape d'affichage du second message.

10. Procédé selon la revendication 7, comprenant en outre les étapes consistant à : au moins afficher un troisième message sur l'afficheur à cristaux liquides, si ledit excès de chaleur indique que la pièce/la lame est chaude, ou arrêter le système chirurgical à ultrasons.

11. Procédé selon la revendication 10, dans lequel l'étape d'affichage du troisième message comprend l'affichage d'un message « pièce chaude » sur l'afficheur à cristaux liquides.
